(19) 

Europäisches
Patentamt
European
Patent Office
Office européen
des brevets

(11) **EP 4 779 307 A1**

(12) **EUROPEAN PATENT APPLICATION**
published in accordance with Art. 153(4) EPC

(43) Date of publication:
**22.07.2026   Bulletin 2026/30**

(21) Application number: **24873860.1**

(22) Date of filing: **27.12.2024**

(51) International Patent Classification (IPC):
**G01N 33/543** (2006.01)   **G01N 33/569** (2006.01)

(52) Cooperative Patent Classification (CPC):
**G01N 33/54389; G01N 33/56983;** G01N 2333/11;
G01N 2333/165

(86) International application number:
**PCT/CN2024/143209**

(87) International publication number:
**WO 2026/113102 (04.06.2026 Gazette 2026/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **29.11.2024   CN 202411737690**

(71) Applicant: **Bioteke Corporation (Wuxi) Co., Ltd.
Wuxi, Jiangsu 214100 (CN)**

(72) Inventors:
• **LI, Pengcheng**
 **Wuxi, Jiangsu 214100 (CN)**
• **ZENG, Xiaoqiong**
 **Wuxi, Jiangsu 214100 (CN)**
• **ZHOU, Zhitu**
 **Wuxi, Jiangsu 214100 (CN)**

(74) Representative: **Angerhausen, Christoph
Boehmert & Boehmert
Anwaltspartnerschaft mbB
Pettenkoferstrasse 22
80336 München (DE)**

(54)   **TEST STRIP FOR COMBINED IMMUNOCHROMATOGRAPHIC DETECTION OF MULTIPLE
RESPIRATORY PATHOGENIC MICROORGANISMS**

(57)   Provided is a combined immunochromato-graphic test strip for detection of multiple respiratory pathogenic microorganisms, belonging to the technical field of in vitro diagnostic reagents. Provided is a test strip for detection of a H-shaped structure, including test strips vertically placed on the left and right, and a horizontally placed sampling strip. A middle part of each of a test strip 1 and a test strip 2 is provided with a sampling zone, with the sampling zone as the center, a conjugate zone, a reaction zone and an absorption zone are symmetrically overlapped in sequence above and below. One end of the sampling strip is overlapped with the sampling zone of the test strip 1, and the other end of the sampling strip is overlapped with the sampling zone of the test strip 2. The test strip for detection has the characteristics of high detection rate, avoidance of cross reaction and simple operation, and has obvious advantages compared with the traditional test strip.

**FIG. 1**

**Description**

**CROSS-REFERENCE TO RELATED APPLICATION**

This patent application claims the priority of Chinese Patent Application No. 202411737690.9 filed with the China National Intellectual Property Administration on Nov 29, 2024, and entitled "Combined immunochromatographic test strip for detection of multiple respiratory pathogenic microorganisms", the entire content of which is incorporated by reference in the present application.

**TECHNICAL FIELD**

The present disclosure belongs to the technical field of in vitro diagnostic reagents, and in particular to a combined immunochromatographic test strip for detection of multiple respiratory pathogenic microorganisms.

**BACKGROUND**

There is relatively high incidence of respiratory infections, and the respiratory infections caused by respiratory viruses such as influenza viruses (including influenza A virus and influenza B virus) and human respiratory syncytial virus occur mainly among young children, pregnant women, the elderly, and patients with other comorbidities. Influenza virus and human respiratory syncytial virus are also main pathogens of acquired pneumonia. In addition to influenza virus and human respiratory syncytial virus, the clinically common viruses that may cause respiratory infections include human metapneumovirus, rhinovirus, parainfluenza virus, coronavirus, adenovirus and bocavirus. In addition, other common pathogenic microorganisms in respiratory tract also include mycoplasma pneumoniae, and streptococcus pneumoniae.
At present, the commonly used methods for detecting respiratory viruses, mycoplasma pneumoniae and streptococcus pneumoniae are mainly detected separately to prevent cross reaction during pathogen detection from affecting the accuracy of results. However, detected separately undoubtedly has a high demand for testing samples, and requires multiple sampling. Moreover, the increase of the number of sampling leads to the shortage of sample treatment solution, making it necessary to increase the volume of sample treatment solution, which will undoubtedly cause the decrease of the sample concentration, reducing the detection rate reduced.

**SUMMARY**

In view of this, an objective of the present disclosure is to provide a combined immunochromatographic test strip for detection of multiple respiratory pathogenic microorganisms. The test strips are arranged as an H-shaped structure, sample application zones are arranged at symmetrical center points of the H-shaped structure, and meanwhile, in the H-shaped structure, two conjugate zones, two reaction zones and two water absorbent zones are symmetrically distributed on each of the left and right test strips with the sample application zone as the middle zone, respectively, thus implementing combined detection of multiple respiratory pathogens, and the simultaneous detection of ten pathogenic microorganisms can be implemented with only one sample application.
The present disclosure provides a combined immunochromatographic test strip for detection of multiple respiratory pathogenic microorganisms, including a test strip 1 and a test strip 2 which are both placed vertically, and a horizontally placed sample application strip.
A middle part of each of the test strip 1 and the test strip 2 is provided with a sample application zone, and with the sample application zone as the center, conjugate zones, reaction zones and absorption zones are sequentially and symmetrically overlapped above and below the sample application zone. One end of the sample application strip is overlapped with the sample application zone of the test strip 1, and the other end of the sample application strip is overlapped with the sample application zone of the test strip 2.
Each conjugate zone is loaded with at least one primary antibody labeled with a latex microsphere; each reaction zone is provided with at least one test line and a control line. The test line is coated with a secondary antibody; the primary antibody and the secondary antibody can specifically bind to the same respiratory pathogenic microorganism.
In some embodiments, the test strip 1 is overlapped with a first absorbent zone, a first reaction zone, a first conjugate zone, a first sample application zone, a second conjugate zone, a second reaction zone, and a second absorbent zone in turn from top to bottom.
The test strip 2 is overlapped with a third absorbent zone, a third reaction zone, a third conjugate zone, a second sample application zone, a fourth conjugate zone, a fourth reaction zone and a fourth absorbent zone in turn from top to bottom.
The first conjugate zone, the second conjugate zone, the third conjugate zone and the fourth conjugate zone are loaded with different types of primary antibodies against respiratory pathogenic microorganisms labeled with latex microspheres, respectively.

Each of the first reaction zone, the second reaction zone, the third reaction zone and the fourth reaction zone is provided with a control line and test lines, where the number of the test lines is the same as that of primary antibodies loaded on the conjugate zone at the same end of the test strip; and each test line is coated with a secondary antibody against the respiratory pathogenic microorganism.

In some embodiments, on each conjugate zone, a loading mass concentration of the primary antibody against the respiratory pathogenic microorganism labeled with the latex microsphere is independently 0.3%-0.4%.

In some embodiments, a particle size of the latex microsphere ranges from 300 nm to 400 nm.

In some embodiments, on each test line, a coating concentration of secondary antibody against the respiratory pathogenic microorganism is independently 0.5-2.0 mg/mL.

In some embodiments, each control line is coated with goat anti-mouse IgG.

In some embodiments, a coating concentration of the goat anti-mouse IgG ranges from 0.8 mg/mL to 1.5 mg/mL.

In some embodiments, a sample application pad in the sample application zone on each of the test strip 1 and the test strip 2 and a sample application pad on the sampling strip are pre-treated with a first treatment solution.

The first treatment solution is an aqueous solution containing 0.5% to 1% by mass of polyvinylpyrrolidone, 0.5% to 1% by mass of casein, 0.5% to 0.8% by volume of Tween-20, and 0.8% to 1.2% by mass of Tris.

In some embodiments, the first conjugate zone is coated with a primary antibody against influenza B virus labeled with a blue latex microsphere, a primary antibody against influenza A virus labeled with a blue latex microsphere, and a primary antibody against novel coronavirus labeled with a red latex microsphere. The second conjugate zone is coated with a primary antibody against mycoplasma pneumoniae labeled with the red latex microsphere, a primary antibody against respiratory adenovirus labeled with the blue latex microsphere, and a primary antibody against respiratory syncytial virus labeled with the blue latex microsphere. The third conjugate zone is coated with a primary antibody against rhinovirus labeled with the blue latex microsphere, and a primary antibody against parainfluenza virus labeled with the red latex microsphere. The fourth conjugate zone is coated with a primary antibody against group A streptococcus labeled with the red latex microsphere, and a primary antibody against streptococcus pneumoniae labeled with the blue latex microsphere.

The first reaction zone is provided with three test lines and one first control line, the three test lines are coated with a secondary antibody against influenza B virus, a secondary antibody against influenza A virus, and a secondary antibody against novel coronavirus, respectively.

The second reaction zone is provided with three test lines and one second control line, the three test lines are coated with a secondary antibody against mycoplasma pneumoniae, a secondary antibody against respiratory adenovirus, and a secondary antibody against respiratory syncytial virus, respectively.

The third reaction zone is provided with two test lines and one third control line, and the two test lines are coated with a secondary antibody against rhinovirus, and a secondary antibody against parainfluenza virus, respectively.

The fourth reaction zone is provided with two test lines and one fourth control line, and the two test lines are coated with a secondary antibody against group A streptococcus, and a secondary antibody against streptococcus pneumoniae, respectively.

The present disclosure provides a combined immunochromatographic test strip card for detection of multiple respiratory pathogenic microorganisms, including the test strip for detection, a card slot base plate, and a cover plate.

A shape of a card slot on the card slot base plate is in fit with a structure of the test strip for detection to fix the test strip for detection.

The cover plate is provided with a hollow sample well and a plurality of observation windows.

The sample well is located above the sampling strip; an observation window is arranged above each reaction zone for observing a test result.

The present disclosure provides a combined immunochromatographic test strip for detection of multiple respiratory pathogenic microorganisms, including a test strip 1 and a test strip 2 which are both placed vertically, and a horizontally placed sampling strip. A middle part of each of the test strip 1 and the test strip 2 is provided with a sampling zone, and with the sampling zone as the center, conjugate zones, reaction zones and absorption zones are sequentially and symmetrically overlapped above and below. One end of the sampling strip is overlapped with the sampling zone of the test strip 1, and the other end of the sampling strip is overlapped with the sampling zone of the test strip 2. Each conjugate zone is loaded with at least one primary antibody labeled with a latex microsphere. Each reaction zone is provided with at least one test line and a control line. The test line is coated with a secondary antibody. The primary antibody and the secondary antibody can specifically bind to the same respiratory pathogenic microorganism. The test strip for detection has the characteristics of high detection rate. In the traditional detection, as the target increases, the sample needs to be applied multiple times, which leads to the shortage of sample treatment solution, making it necessary to increase the volume of sample treatment solution, which will undoubtedly cause the decrease of the sample concentration. The H-shaped test strip for detection provided by the present disclosure can avoid the above problem well. The test strip for detection can also avoid cross reaction: some pathogens with high similarity will cross to a certain extent during detection, which cannot be completely avoided in the previous bidirectional chromatography scheme, and can be improved only by optimizing the process. The test strip provided by the present disclosure has an H-shaped structure, which can avoid crossing by physical barriers. In addition, the test strip for detection provided by the present disclosure has the characteristics of simple operation. Compared with the problem that the traditional detection faces multiple sampling with the increase of the target, the H-shaped test strip only requires once sampling, making the operation faster and simpler.

## BRIEF DESCRIPTION OF THE DRAWINGS

FIG. 1 is a diagram of a front structure of a cover plate of a test strip card for detection of the present disclosure;
FIG. 2 is a diagram of a back structure of a cover plate of a test strip card for detection of the present disclosure, where 1 is a boss, and 2 is a raised point;
FIG. 3 is a structural diagram of a card slot base plate of a test strip card for detection of the present disclosure;

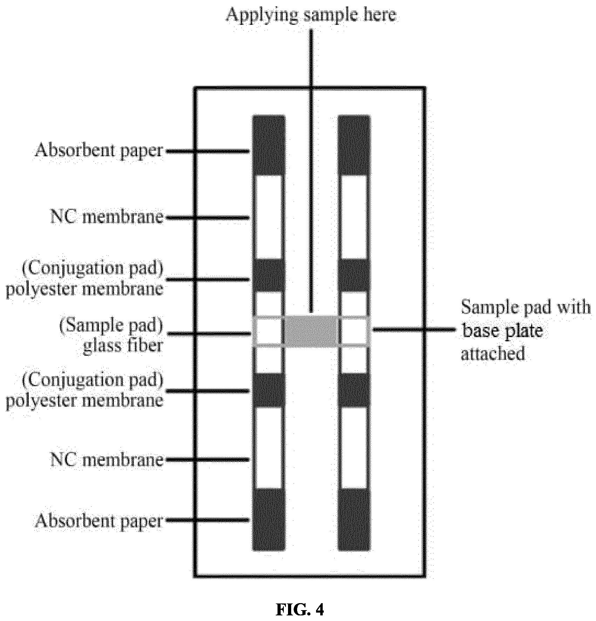

FIG. 4 is a structural diagram of an H-shaped test strip for detection of the present disclosure.

## DETAILED DESCRIPTION OF THE EMBODIMENTS

The present disclosure provides an H-shaped combined immunochromatographic test strip for detection of multiple respiratory pathogenic microorganisms, including a test strip 1 and a test strip 2 which are both placed vertically, and a horizontally placed sampling strip. A middle part of each of the test strip 1 and the test strip 2 is provided with a sampling zone, and with the sampling zone as the center, conjugate zones, reaction zones and absorption zones are sequentially and symmetrically overlapped above and below. One end of the sampling strip is overlapped with the sampling zone of the test strip 1, and the other end of the sampling strip is overlapped with the sampling zone of the test strip 2. Each conjugate zone is loaded with at least one primary antibody labeled with a latex microsphere. Each reaction zone is provided with at least one test line and a control line. The test line is coated with a secondary antibody. The primary antibody and the secondary antibody can specifically bind to the same respiratory pathogenic microorganism.

In the present disclosure, the test strip for detection includes a base card, and a sampling zone, a conjugate zone, a reaction zone and an absorbent zone fixed to the base card. In some embodiments, the base card includes a polyvinyl chloride (PVC) base card with an adhesive on one side. In some embodiments, the test strip 1 is overlapped with a first absorbent zone, a first reaction zone, a first conjugate zone, a first sampling zone, a second conjugate zone, a second reaction zone and a second absorbent zone in turn from top to bottom. In some embodiments, the test strip 2 is overlapped with a third absorbent zone, a third reaction zone, a third conjugate zone, a second sampling zone, a fourth conjugate zone, a fourth reaction zone and a fourth absorbent zone in turn from top to bottom. In some embodiments, each of the first conjugate zone, the second conjugate zone, the third conjugate zone and the fourth conjugate zone is loaded with at least one primary antibody against different respiratory pathogenic microorganisms labeled with latex microspheres. Each of the first reaction zone, the second reaction zone, the third reaction zone and the fourth reaction zone is provided with a control line, and test lines, where the number of the test lines is the same as that of the primary antibodies loaded on the conjugate zone at the same end of the test strip. The test lines with the same number as the primary antibodies loaded on the conjugate zone at the same end of the test strip mean that the number of the test lines coated on the first reaction zone is the same as that of the primary antibodies against respiratory pathogenic microorganisms labeled with latex microspheres loaded in the first conjugate zone, and the number of the test lines coated on the second reaction zone is the same as that of the primary antibodies against respiratory pathogenic microorganisms labeled with the latex microspheres loaded in the second conjugate zone, and so on. The test line is coated with a secondary antibody against respiratory pathogenic microorganism. The H-shaped test strip for detection is provided with sample spotting zones at the center symmetrical points, such a structure is conducive to implementing simultaneous detection of multiple pathogenic microorganisms by one sampling, and the demand for the sample size can be reduced while greatly simplifying the operation, thus meeting the detection demand of multiple targets with low sample size. In some embodiments, a material at each conjugate zone includes a polyester membrane, and/or glass fiber. A material at each reaction zone preferably includes a cellulose nitrate membrane. In some embodiments, a material at each absorbent zone includes filter paper. In some embodiments, a material at each sampling zone includes the polyester membrane, and/or the glass fiber.

In some embodiments of the present disclosure, a loading mass concentration of a primary antibody against respiratory pathogenic microorganism labeled with the latex microsphere loaded on each conjugate zone independently ranges from 0.3% to 0.4%, preferably 0.3%. In some embodiments, a solvent of the primary antibody against respiratory pathogenic microorganism labeled with the latex microsphere is a Tris-Casein buffer solution. In some embodiments, the Tris-Casein buffer solution is an aqueous solution containing 1.5 M Tris-HCl and 0.5% of Casein, with a pH of 8.0-8.2. There is no special limitation on the method for preparing the primary antibody against respiratory pathogenic microorganisms labeled with latex microspheres, and it is only necessary to use the method of labeling antibodies with the latex microspheres which is well known in the art. The primary antibodies against different types of respiratory pathogenic microorganisms can be labeled with latex microspheres of different colors. In some embodiments, the color of the latex microsphere includes red and blue. In some embodiments,, a particle size of the latex microsphere ranges from 300 nm to 400 nm.

In some embodiments of the present disclosure, a conjugate pad in each conjugate zone is a polyester membrane. In some embodiments, the conjugate pad is pretreated with a second treatment solution, which is conducive to improving the solubility of the primary antibody against respiratory pathogenic microorganisms labeled with latex microsphere loaded in the conjugate zone, and reducing the residue of the primary antibody on the conjugate pad. In some embodiments, the second treatment solution is an aqueous containing the following components: 8%-12% by mass of 3-(N-morpholino)-2-hydroxypropanesulfonic acid, 4%-6% by mass of casein, 0.4%-0.6% by volume of Tween-20 and 0.9%-1.1% by volume of Proclin 300, or an aqueous solution containing the following components: 10% by mass of 3-(N-morpholino)-2-hydroxypropanesulfonic acid, 5% by mass of casein, 0.5% by volume of Tween-20 and 1% by volume of Proclin 300.

In some embodiments of the present disclosure, a coating concentration of the secondary antibody against the respiratory pathogenic microorganism ranges from 0.5 mg/mL to 2.0 mg/mL, preferably ranging from 0.8 mg/ml to 1.5 mg/ml, and most preferably 1.0 mg/mL. In some embodiments, the control line is coated with goat anti-mouse IgG. In some embodiments, a coating concentration of the goat anti-mouse IgG ranges from 0.8 mg/mL to 1.5 mg/mL, which may be 1.0 mg/mL. In some embodiments, a solvent of the secondary antibody against the respiratory pathogenic microorganism includes 0.01 M PBS solution.

In some embodiments of the present disclosure, a sampling pad in the sampling zone on each of the test strip 1 and the test strip 2 and a sampling pad on the sampling strip are pre-treated with a first treatment solution. In some embodiments, the first treatment solution is an aqueous solution containing the following components: 0.5%-1% by mass of polyvinylpyrrolidone, 0.5%-1% by mass of casein, 0.5%-0.8% by volume of Tween-20, and 0.8%-1.2% by mass of Tris, which may be an aqueous solution containing the following components: 0.5% by mass of polyvinylpyrrolidone, 0.5% by mass of casein, 0.5% by volume of Tween-20, and 1.0% by mass of Tris. The treatment of the sampling pad with the treatment solution is conducive to eliminating the difference between different pH values of the samples and changing the flow of the samples and improving a reaction system, thus improving the detection sensitivity and eliminating nonspecific binding.

In an embodiment of the present disclosure, when the respiratory pathogenic microorganisms include influenza B virus, influenza A virus, novel coronavirus, mycoplasma pneumoniae, respiratory adenovirus, respiratory syncytial virus, rhinovirus, parainfluenza virus, streptococcus pneumoniae, and group A streptococcus, the first conjugate zone is coated with a primary antibody against influenza B virus labeled with a blue latex microsphere, a primary antibody against influenza A virus labeled with a blue latex microsphere, and a primary antibody against novel coronavirus labeled with a red latex microsphere; the second conjugate zone is coated with a primary antibody against mycoplasma pneumoniae labeled with the red latex microsphere, a primary antibody against respiratory adenovirus labeled with the blue latex microsphere, and a primary antibody against respiratory syncytial virus labeled with the blue latex microsphere; the third conjugate zone is coated with a primary antibody against rhinovirus labeled with the blue latex microsphere, and a primary antibody against parainfluenza virus labeled with the red latex microsphere; and the fourth conjugate zone is coated with a primary antibody against group A streptococcus labeled with the red latex microsphere, and a primary antibody against streptococcus pneumoniae labeled with the blue latex microsphere. The first reaction zone is provided with three test lines and one first control line, the three test lines are coated with a secondary antibody against influenza B virus, a secondary antibody against influenza A virus, and a secondary antibody against novel coronavirus, respectively. The second reaction zone is provided with three test lines and one second control line, the three test lines are coated with a secondary antibody against mycoplasma pneumoniae, a secondary antibody against respiratory adenovirus, and a secondary antibody against respiratory syncytial virus, respectively. The third reaction zone is provided with two test lines and one third control line, the two test lines are coated with a secondary antibody against rhinovirus, and a secondary antibody against parainfluenza virus, respectively. The fourth reaction zone is provided with two test lines and one fourth control line, and the two test lines are coated with a secondary antibody against group A streptococcus, and a secondary antibody against streptococcus pneumoniae, respectively. The primary antibody against novel coronavirus is purchased from Beijing Baixinyi Biotechnology Co., Ltd., with catalog number of 2020TN15. The primary antibody against influenza A virus is purchased from Shenzhen AIVD Biotechnology Co., Ltd., with catalog number of ABFLUA05. The primary antibody against influenza B virus is purchased from Nanjing Santa Scott Biotechnology Co., Ltd., with catalog number of S-1TFB2. The primary antibody against respiratory syncytial virus is purchased from Feipeng Biotechnology Co., Ltd., Guangzhou Branch, with catalog number of RSV-REAB-G1-012. The primary antibody against adenovirus is purchased from Beijing Yinghe Lingyuan Biotechnology Co., Ltd., with catalog number of Baa001. The primary antibody against mycoplasma pneumoniae is purchased from Xiamen Huasheng Era Biotechnology Co., Ltd, with catalog number of HSA02. The primary antibody against parainfluenza virus is purchased from Xinxin Biotechnology Co., Ltd. (ACTHTEAM, LLC), with catalog number of ACT-mAb-PIVS1-002. The primary antibody against group A streptococcus is purchased from Shandong Zuocon Biotechnology Co., Ltd., with catalog number of STA-Ab1. The primary antibody against rhinovirus is purchased from Xinxin Biotechnology Co., Ltd. (ACTHTEAM, LLC), with catalog number of ACT-mAb-RhV-002. The primary antibody against streptococcus pneumoniae is purchased from Xinxin Biotechnology Co., Ltd. (ACTHTEAM, LLC), with catalog number of ACT-mAb-SP-003. The secondary antibody against novel coronavirus is purchased from Beijing Baixinyi Biotechnology Co., Ltd., with catalog number of 2020TN8. The secondary antibody against influenza A virus is purchased from Shenzhen AIVD Biotechnology Co., Ltd., with catalog number of ABFLUA06. The secondary antibody against influenza B virus is purchased from Nanjing Santa Scott Biotechnology Co., Ltd., with catalog number of S-1TFB1. The secondary antibody against respiratory syncytial virus is purchased from Feipeng Biotechnology Co., Ltd., Guangzhou Branch, with catalog number of RSV-REAB-G1-011. The secondary antibody against adenovirus is purchased from Beijing Yinghe Lingyuan Biotechnology Co., Ltd., with catalog number of Baa001-2. The secondary antibody against mycoplasma pneumoniae is purchased from Xiamen Huasheng Era Biotechnology Co., Ltd, with catalog number of HSA01. The secondary antibody against parainfluenza virus is purchased from Xinxin Biotechnology Co., Ltd. (ACTHTEAM, LLC), with catalog number of ACT-mAb-PIVS1-001. The secondary antibody against group A streptococcus is purchased from Shandong Zuocon Biotechnology Co., Ltd., with catalog number of STA-Ab1. The secondary antibody against rhinovirus is purchased from Xinxin Biotechnology Co., Ltd. (ACTHTEAM, LLC), with catalog number of ACT-mAb-RhV-001. The secondary antibody against streptococcus pneumoniae is purchased from Xinxin Biotechnology Co., Ltd.(ACTHTEAM, LLC), with catalog number of ACT-mAb-SP-001.

The present disclosure provides a combined immunochromatographic test strip card for detection of multiple respiratory pathogenic microorganisms, including a test strip for detection, a card slot base plate, and a cover plate. A shape of a card slot on the card slot base plate is in fit with a structure of the test strip to fix the test strip for detection. The cover plate is provided with a hollow sample well, and four observation windows. The sample well is located above a vertical direction of the sampling strip. The four observation windows are located above vertical directions of a first reaction zone, a second reaction zone, a third reaction zone, and a fourth reaction zone, respectively.

In the present disclosure, the card slot base plate and the cover plate have the consistent length and width. The periphery of a back side of the cover plate is distributed with multiple raised points, while the corresponding positions on a front side of the card slot base plate are distributed with multiple recessed points. The card slot base plate and the cover plate are sealed and fastened by inserting the raised points into the recessed points. Multiple linear bosses are distributed on the back side of the cover plate, and are configured to secure the test strip in a groove of the card slot base plate. The bosses are distributed between two vertically distributed observation windows. In some embodiments, the number of the bosses ranges from 2 to 4, which may be 3. In some embodiments, the raised point, the boss and the cover plate are integrally formed. In an embodiment of the present disclosure, a housing formed by snapping the cover plate onto the card slot base plate has an overall length of 118 mm, and a width of 35.7 mm. A card slot in the cart slot base plate has a width of 4.6 mm. The cover plate has a height of 2.1 mm, the boss in the cover plate has a height of 1.85 mm, and a length from the boss close to the reaction zone to a nearest edge of the observation window is 1.8 mm.

In some embodiments of the present disclosure, a method for preparing the test strip card for detection includes the following steps:

pretreating a sampling pad in a sampling zone in each of a test strip 1 and a test strip 2 as well as a sampling pad on a sampling strip with a first treatment solution, and drying to obtain pre-treated sampling pads;

spraying primary antibodies against different respiratory pathogenic microorganisms labeled with latex microspheres of different colors on a conjugate pad directly or in a hybrid manner, and drying to obtain a conjugate pad loaded with the primary antibodies labeled with the latex microspheres;

respectively spraying corresponding second antibodies against different types of respiratory pathogenic microorganisms on a nitrocellulose membrane to form test lines, and spraying mouse anti-IgG on the nitrocellulose membrane to form a control line, thus obtaining the nitrocellulose membrane coated with different second antibodies;

overlapping and assembling the sampling pad, the conjugate pad loaded with the primary antibodies labeled with the latex microsphere, the nitrocellulose membrane coated with different second antibodies and an absorbent pad to obtain a test board, and cutting the test board into strips to obtain test strips for detection; and

placing the test strips for detection on a card slot base plate, overlapping one end of a sampling strip on a sampling zone of the test strip 1, and the other end of the sampling strip on a sampling zone of the test strip 2, and snapping on a cover plate to obtain a test strip card for detection.

Alternatively, the sampling pad and the sampling strip are integrally formed. During assembly, after the nitrocellulose membranes are symmetrically pasted on the base card, the conjugate pads and the water absorbent pads are pasted on the base card, then the base card is cut into strips, and the integrally formed sampling pad is pasted on each strip to obtain a test strip for detection. The obtained test strip for detection is placed on the card slot base plate, and then the cover plate is snapped on the card slot base plate to obtain the test strip card for detection.

In the present disclosure, an overlapping distance of the conjugate pad and the nitrocellulose membrane may range from 1.3 mm to 1.7 mm, which may also be 1.5 mm. An overlapping distance of a sample pad and the conjugate pad may range from 2 mm to 2.7 mm, which may also be 2.5 mm. An overlapping distance of an absorbent paper and the nitrocellulose membrane may range from 2.8 mm to 3.2 mm, which may also be 3 mm.

In some embodiments of the present disclosure, a detection method of the test strip card for detection is based on a double-antibody sandwich principle, specifically including the following steps:

treating a sample with a sample extraction solution to obtain a treated sample;
dropping the treated sample into a sample well of the test strip card for detection, standing for observation, and determining whether there is infection of a target pathogenic microorganism in the sample according to color development at the observation window:
when a control line in the observation window develops color and a test line develops color at the same time, indicating that the sample is positive;
when the control line in the observation window develops color but the test line does not develop color, indicating that the sample is negative; and
when the control line in the observation window does not develop, whether the test line develops color or not, the detection is repeated.

In some embodiments of the present disclosure, the sample extraction solution is normal saline containing 0.5% by volume of Tween-20, and 0.5% by volume of Triton X-100. In some embodiments, a collection method of the treated sample is nasal fluid collection.

In an embodiment of the present disclosure, the test strip card for detection is used for detection, the results show that compared with the conventional test strip card for detection, the detection efficiency is high, the number of sampling is less, the color development is more significant, and the color development for the low-concentration sample is stronger, indicating that the test strip card for detection provided by the present disclosure has higher detection sensitivity.

In the following, a combined immunochromatographic test strip for detection for multiple respiratory pathogenic microorganisms provided by the present disclosure will be described in detail with examples, but they cannot be understood as limiting the scope of protection of the present disclosure.

**Example 1**

A structure of a combined immunochromatographic test strip for detection of multiple respiratory pathogenic microorganisms and a preparation method for the combined immunochromatographic test strip for detection of multiple respiratory pathogenic microorganisms

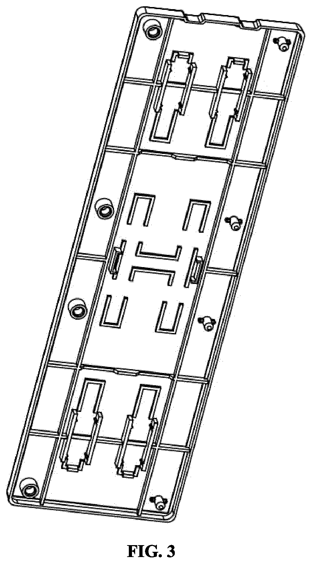

1. The test strip for detection had an H-shaped structure (shown in FIG. 3), in which a test strip 1 was sequentially overlapped with a first water absorbent zone, a first reaction zone, a first conjugate zone, a first sampling zone, a second conjugate zone, a second reaction zone and a second water absorbent zone from top to bottom, and a test strip 2 was sequentially overlapped with a third water absorbent zone, a third reaction zone, a third conjugate zone, a second sampling zone, a fourth conjugate zone, a fourth reaction zone and a fourth water absorbent zone from top to bottom, and both ends of a sampling strip were respectively overlapped with the sampling zones of the test strip 1 and the sampling zone of the test strip 2. When the target pathogenic microorganisms included influenza B virus, influenza A virus, novel coronavirus, mycoplasma pneumoniae, respiratory adenovirus, respiratory syncytial virus, rhinovirus, parainfluenza virus, streptococcus pneumoniae and group A streptococcus, the first conjugate zone was coated with a primary antibody against influenza B virus labeled with a blue latex microsphere, a primary antibody against influenza A virus labeled with a blue latex microsphere, and a primary antibody against novel coronavirus labeled with a red latex microsphere; the second conjugate zone was coated with a primary antibody against mycoplasma pneumoniae labeled with the red latex microsphere, a primary antibody against respiratory adenovirus labeled with the blue latex microsphere, and a primary antibody against respiratory syncytial virus labeled with the blue latex microsphere; the third conjugate zone was coated with a primary antibody against rhinovirus labeled with the blue latex microsphere, and a primary antibody against parainfluenza virus labeled with the red latex microsphere; and the fourth conjugate zone was coated with a primary antibody against group A streptococcus labeled with the red latex microsphere, and a primary antibody against streptococcus pneumoniae labeled with the blue latex microsphere. The first reaction zone was provided with three test lines and one first control line, the three test lines included a first test line, a second test line and a third test line distributed in sequence from bottom to top, where the first test line was coated with a secondary antibody against influenza B virus, the second test line was coated with a secondary antibody against influenza A virus, and the third test line was coated with a secondary antibody against novel coronavirus. The second reaction zone was provided with three test lines and one second control line, the three test lines included a first test line, a second test line and a third test line distributed in sequence from bottom to top, where the first test line was coated with a secondary antibody against mycoplasma pneumoniae, the second test line was coated with a secondary antibody against respiratory adenovirus, and the third test line was coated with a secondary antibody against respiratory syncytial virus. The third reaction zone was provided with two test lines and one third control line, the two test lines included a first test line and a second test line arranged in sequence form bottom to top, where the first test line was coated with a secondary antibody against rhinovirus, and the second test line was coated with a secondary antibody against parainfluenza virus. The fourth reaction zone was provided with two test lines and one fourth control line, the two test lines included a first test line and a second test line arranged in sequence form bottom to top, the first test line was coated with a secondary antibody against group A streptococcus, and the second test line was coated with a secondary antibody against streptococcus pneumoniae.

## 2. Preparation method

2.1. A preparation method for a primary antibody against influenza B virus labeled with a blue latex microsphere was taken as an example to illustrate the preparation method for the primary antibody against pathogenic microorganism labeled with latex microsphere.

### 2.1.1. Cleaning of the latex microsphere

(1) the required volume of the blue latex microspheres were calculated according to Formula I:

$$\text{the volume of blue latex microspheres} = \text{final volume} \times 1\% \div 4\% \text{ (Formula I)};$$

where the final volume was 0.6 mL during actual preparation;
(2) the required volume of diluted blue latex microspheres were calculated to be added to 1% borate buffer solution (pH is 8.0±0.1) according to Formula II:

$$\text{the volume of borate buffer solution} = \text{total volume} - \text{the volume of blue latex microsphere}$$

$$\text{(Formula II)};$$

where the final volume was 1.5 mL during actual preparation;
(3) the blue latex microspheres were added to the borate buffer solution, and fully mixed, centrifuged at 14,000 rpm for 15 min with a centrifuge, and a resulting supernatant is removed with a pipette;
(4) the blue latex microspheres were cleaned again, the borate buffer solution was added into the blue latex microspheres, where the total sonication time was 20 seconds with a pulse cycle of 0.3 seconds on and 0.2 seconds off;

2.1.2. activation of the latex microspheres, and preparation of an EDC solution (the prepared EDC solution must be used up within 10 minutes):

(1) the volume of EDC was calculated according to Formula III, where the minimum volume was 0.5 mL:

$$\text{the volume of EDC} = \text{the final solution of solution} \times 0.07 \text{ mL/ml (Formula III)};$$

(2) the mass of EDC was calculated according to Formula IV, and weighed:

$$\text{the mass of EDC} = \text{the volume of EDC} \times 10 \text{ mg/mL (Formula IV)};$$

(3) the volume of the required water was calculated according to Formula V:

$$\text{the volume of water} = \text{the volume of EDC} \div 10 \text{ mg/mL (Formula V)};$$

2.1.3. preparation of a primary antibody against influenza B virus labeled with blue latex microsphere:

(1) the required primary antibody against influenza B virus was calculated according to Formula VI:

the mass of the primary antibody to be labeled= final volume ×labeling ratio (1.0 mg/mL) ÷stock concentration(Formula VI);  (Formula VI);

where the final volume was 0.6 mL during actual preparation;
(2) the required primary antibody against influenza B virus was weighed, added into the cleaned latex microspheres, the required volume of borate buffer solution was added immediately, and then the required volume of EDC was added, and a resulting product was shook and mixed uniformly for 1 min;
(3) a resulting product was stirred at a room temperature overnight to obtain a latex antibody solution;

2.1.4. preparation of an ethanolamine solution

(1) the required amount of ethanolamine was calculated according to Formula VII, added into the latex antibody solution, and mixed for 30 min:

$$\text{the volume of ethanolamine=the final volume of latex antibody solution} \times 0.14 \text{ mL/ml}$$

(Formula VII);

(2) a resulting product was centrifuged at 14,000 rpm for 15 min with a centrifuge, and the supernatant was removed;

2.1.5. preparation of a Tris-Casein buffer solution

(1) the Tris-Casein buffer solution was added into the primary antibody labeled with latex microsphere for sonication, where the total sonication time was 20 seconds with a pulse cycle of 0.3 seconds on and 0.2 seconds off; then mixed uniformly for 4 h;
(2) a resulting product was centrifuged at 14,000 rpm for 15 min with a centrifuge, and the supernatant was removed ;
the Tris-Casein buffer solution (1.5 M Tris-HCl, 0.5% of Casein, pH of 8.0-8.2) was added into the primary antibody labeled with latex microsphere after cleaning, and sonication water bath was performed for 15 min to obtain a primary antibody solution against influenza B virus labeled with latex microsphere.

2.2. Preparation of a conjugate pad is as follows:

2.2.1. pretreating of a polyester membrane was conducted as follows:

(1) an untreated polyester pad was soaked in a preparation box filled with a treatment solution, ensuring that the polyester was completely immersed in the treatment solution and the liquid level higher than that of the polyester pad;
(2) the preparation box was placed in a shaker to shake slowly for 5 h;
(3) a single sheet of polyester pad was picked up to drain excess treatment solution until the treatment solution was in droplet form;
(4) placing the drained polyester pad in a drying room or a drying oven for drying, where drying is carried out at a temperature of 40°C and relative humidity of no more than 20% for 12 h;

where a formula of the treatment solution was as follows: 10% by mass of 3-(N-morpholino)-2-hydroxypropanesulfonic acid, 5% by mass of casein, 0.5% by volume of Tween-20, and 1% by volume of Proclin 300; and a concentration of the prepared primary antibody solution against pathogenic microorganism labeled with each latex microsphere was adjusted to 0.3% with a solvent to obtain a primary antibody working solution labeled with the latex microsphere; the primary antibodies working solution labeled with specific types of latex microspheres were mixed and sprayed on the pretreated polyester membrane, and then dried to obtain the polyester membrane loaded with the primary antibody labeled with the latex microsphere.

2.3. A preparation method for a nitrocellulose membrane coated with a secondary antibody.

a secondary antibody specific to the pathogenic microorganism with a concentration of 1 mg/mL and an anti-mouse IgG antibody working solution with a concentration of 1.0 mg/mL was striped on the nitrocellulose membrane with a striping instrument, with a coating amount of 30 μL/30 cm, and the nitrocellulose membrane was dried to obtain the nitrocellulose membrane coated with the secondary antibody.

2.4. Treatment of a sample pad

the polyester membrane was soaked in the sample treatment solution for 1 h, and then taken out for drying to obtain a pretreated sample pad, where the sample treatment solution was an aqueous solution containing 0.5% by mass of polyvinylpyrrolidone, 0.5% by mass of casein, 0.5% by volume of Tween-20, and 1.0 % by mass of Tris.

2.5. Assembly and cutting of test strip

the sample pad was attached to the middle of a PVC base card, and the conjugate pads loaded with the primary antibody labeled with the latex microsphere, the nitrocellulose membranes coated with the secondary antibody and absorbent paper was overlapped symmetrically on both sides of the sample pad in a vertical direction, and the assembled card was cut into strips by a strip cutter, with each strip having a width of 4 mm. And the cut test strips according to the type of the detection object was assembled to obtain an H-shaped test strip.

2.6. Preparation of a test strip card for detection

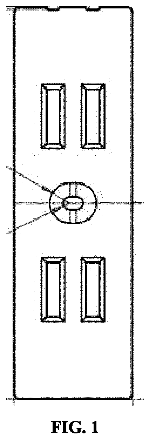

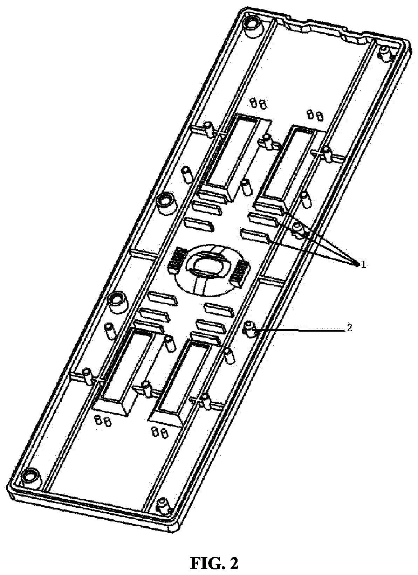

a cover plate (FIG. 1 and FIG. 2) and a card slot base plate (FIG. 3) of a housing were prepared, where the housing formed after the cover plate was snapped on the card slot base plate has an overall length of 118 mm, and a width of 35.7. A card slot in the card slot base plate had a width of 4.6 mm, the cover plate had a height of 2.1 mm, a boss in the cover plate had a height of 1.85 mm, and a length from the boss close to the reaction zone to a nearest edge of an observation window was 1.8 mm. Two test strips were vertically placed on the card slot base plate, and then a sample pad with a small PVC base card was placed in a horizontal direction (i.e., a sampling strip for connecting the test strip 1 and the test strip 2), the sampling strip was constructed to connect the test strip 1 and the test strip 2 and just horizontally placed at a groove in the middle of the card slot base plate to obtain the test strip card for detection.

3. A detection method of the test strip card for detection

a swab containing a collected sample was put into 0.5 ml of sample extraction solution in a sample extraction tube immediately, and a swab head was rotated in the sample extraction solution for at least 30 seconds for uniform mixing; meanwhile, the swab head was squeezed through an outer wall of the sample extraction tube for at least 5 times by hand to ensure that the sample is fully eluted in the sampling tube, and the solution was squeezed from the swab head through the outer wall of the sample extraction tube by hand, then the swab was discarded and the extraction tube was capped;

the treated sample solution was added dropwise into the sample well of the test strip card for detection. After being allowed to stand for 10 min, the test results were observed.

When color development was observed on both the test line and the control line, the sample was interpreted as positive, indicating the presence of infection of the pathogenic microorganism corresponding to the secondary antibody on the test line in the sample.

when the color development was observed on control line and no color development was observed on the test line, the sample was interpreted as negative, indicating the absence of infection of the pathogenic microorganism corresponding to the secondary antibody on the test line in the sample; and

when no color development was observed on control line, the detection was repeated.

## Example 2

Example 1 Clinical sample detection of test strip card for detection

Recombinant protein control products of target pathogenic microorganisms were taken as the sample, the samples were treated according to the method of Example 1, which were respectively diluted twice to obtain sample solutions with concentrations of 10 pg/ml, 20 pg/ml, 40 pg/ml, 80 pg/ml and 160 pg/ml in turn. The sample solutions with different concentrations was added dropwise on the test strip card for detection of Example 1, respectively to observe the color development effects. And meanwhile, a sample suffer solution of an anterior nasal swab of healthy people with negative PCR result was tested as a negative matrix for testing.

The source information of the recombinant protein control products of the target pathogenic microorganisms was shown in Table 1.

**Table 1 List of sources of recombinant protein control products of target pathogenic microorganisms**

| Pathogenic microorganism | Supplier | Protein type | Catalog number |
|---|---|---|---|
| Novel coronavirus | Shenzhen Heavy Bio, Inc | 2019-nCov NP (Nucleoprotein) recombinant antigen (NA238) | HP811-50 |
| Influenza A virus | Shenzhen Heavy Bio, Inc | Eukaryotically expressed influenza A NP protein control | HP809-01 |
| Influenza B virus | Shenzhen Heavy Bio, Inc | Eukaryotically expressed influenza B NP protein control | HP809-02 |
| Respiratory Syncytial virus | Shenzhen Heavy Bio, Inc | Recombinant respiratory syncytial virus (RSV)F protein extracellular fragment | HP812-01 |
| Respiratory adenovirus | Shenzhen Heavy Bio, Inc | Recombinant adenovirus Type 55 Hexon protein | HP818-02 |
| Mycoplasma pneumoniae | Xiamen Winbio Biotechnology Co., Ltd. | Mycoplasma pneumoniae antigen | AGMP007 |
| Parainfluenza virus | Jiangsu East-Mab Biomedical Technology Co., Ltd. | HPIV1 NP (His Tag) | A06104 |
| Group A streptococcus | Wuxi OriGene Technology Co., Ltd. | Strep A protein | DP0038-P1 |
| Rhinovirus | Xiamen OneClone Biotech Inc. | Human rhinovirus (hRHV) recombinant antigen | WKL-H0160 |
| Streptococcus pneumoniae | Xiamen OneClone Biotech Inc. | Streptococcus pneumoniae (PsaA) recombinant antigen | WKL-H0111 |

A same sample solution was used as a test object. The conventional test strip card for detection were used for influenza B virus (Jiangsu BioPerfectus Technologies Co., Ltd., SC10101), influenza A virus (Jiangsu BioPerfectus Technologies Co., Ltd., SC10101), novel coronavirus (Wuhan Easy Diagnosis Biomedicine Co., Ltd., AgH-20A), mycoplasma pneumoniae (Hangzhou GENESIS Biodetection & Biocontrol Co., Ltd., P111005), respiratory adenovirus (Hangzhou GENESIS Biodetection & Biocontrol Co., Ltd., P111004), respiratory syncytial virus (Hangzhou GENESIS Biodetection & Biocontrol Co., Ltd., P111003), rhinovirus (Guangzhou Daan Gene Co., Ltd., RP2020-1), parainfluenza virus (Shandong Kanghua Biotechnology Co., Ltd., k48595), streptococcus pneumoniae (Beijing Beier Bioengineering Co., Ltd., 303633), and Group A streptococcus (Beijing Jinwofu Bioengineering Technology Co., Ltd., 55296).

Table 2 Detection results

| Comparative item | Embodiment 1 Test strip card for detection | | | | | | | | | | Conventional test strip card for detection | | | | | | | | | |
|---|---|---|---|---|---|---|---|---|---|---|---|---|---|---|---|---|---|---|---|---|
| Project efficiency | Novel corona virus | Influenza A virus | Influenza B virus | Syncytial virus | Adenovirus | Mycoplasma pneumoniae | Parainfluenza virus | Group A streptococcus | Rhinovirus | Streptococcus pneumoniae | Novel corona virus | Influenza A virus | Influenza B virus | Syncytial virus | Adenovirus | Mycoplasma pneumoniae | Parainfluenza virus | Group A streptococcus | Rhinovirus | Streptococcus pneumoniae |
| Production efficiency | Good | | | | | | | | | | Bad | | | | | | | | | |
| The number of sampling | 1 time (six drops) | | | | | | | | | | 10 times (30 drops) | | | | | | | | | |

| Color development | Multiple colors | | | | | | | | | | Single color | | | | | | | | | |
|---|---|---|---|---|---|---|---|---|---|---|---|---|---|---|---|---|---|---|---|---|
| Negative matrix | - | - | - | - | - | - | - | - | - | - | - | - | - | - | - | - | - | - | - | - |
| 10pg/ml | + | + | + | + | + | + | + | + | + | + | ± | ± | ± | ± | ± | ± | ± | ± | ± | ± |
| 20pg/ml | ++ | ++ | ++ | ++ | ++ | ++ | ++ | ++ | ++ | ++ | + | + | + | + | + | + | + | + | + | + |
| 40pg/ml | ++ | ++ | ++ | ++ | ++ | ++ | ++ | ++ | ++ | ++ | ++ | ++ | ++ | ++ | ++ | ++ | ++ | ++ | ++ | ++ |
| 80pg/ml | ++ + | ++ + | ++ + | ++ + | ++ + | ++ + | ++ + | ++ + | ++ + | ++ + | ++ + | ++ + | ++ + | ++ + | ++ + | ++ + | ++ + | ++ + | ++ + | ++ + |
| 160 pg/ml (Recombinant protein control product for target pathogens) | ++ + | ++ + | ++ + | ++ + | ++ + | ++ + | ++ + | ++ + | ++ + | ++ + | ++ + | ++ + | ++ + | ++ + | ++ + | ++ + | ++ + | ++ + | ++ + | ++ + |

Noted: "-" means no color development; "±" indicates weakly positive, with the color development intensity weaker than that of "+"; and "+" indicates color development intensities of different test lines. The more the "+", the darker the color of the test line.

As could be seen from Table 2 that compared with the conventional test strip card for detection, in Example 1, the production efficiency was higher, the number of sampling was less, and the color development effect was more obvious and clearer, making the test strip card for detection more user-friendly. By detection of the test strip card for detection provided by this example and the conventional test strip card for detection with the recombinant protein, it was found that the test strip card for detection without the conjugate pad in Example 1 had higher sensitivity than the conventional test strip card for detection, and had stronger color development at a low concentration.

**Comparative example 1**

A test strip card for detection was prepared according to a method in Example 1, and the only difference is that the sample pad was treated with the sample pad treatment solution 2. The sample treatment solution 2 is an 0.8% Tris aqueous solution containing 0.5% by mass of polyvinylpyrrolidone and 1% by mass of bovine serum albumin.

**Table 3 Test results**

| Comparative item | Sample pad after optimization | | | | | | | | | | Sample pad before optimization | | | | | | | | | |
|---|---|---|---|---|---|---|---|---|---|---|---|---|---|---|---|---|---|---|---|---|
| Project efficiency | Novel coronavirus | Influenza A virus | Influenza B virus | Syncytial virus | Adenovirus | Mycoplasma pneumoneum | Parainfluenza virus | Group A streptococc | Rhinovirus | Streptococcus pneumoneu | Novel coronavirus | Influenza A virus | Influenza B virus | Syncytial virus | Adenovirus | Mycoplasma pneumoneum | Parainfluenza virus | Group A streptococc | Rhinovirus | Streptococcus pneumoneu |

| | | | | | oniae | us | moniae | | | | | oniae | us | moniae |
|---|---|---|---|---|---|---|---|---|---|---|---|---|---|---|
| Sensitivity | Good | | | | | | | Bad | | | | | | |
| Flow of solution | Smooth | | | | | | | Slow | | | | | | |
| Negative matrix | - - - - - - - - - - | | | | | | | - - - - - - - - - - | | | | | | |
| 10pg /ml | + + + + + + + + + + | | | | | | | - - - - - - - - - - | | | | | | |
| 20pg /ml | + + + + + + + + + + / + + + + + + + + + + | | | | | | | + + + + - + + + + + | | | | | | |
| 40pg /ml | + + + + + + + + + + / + + + + + + + + + + | | | | | | | + + + + + + + + + + / + + + + + + + + + + | | | | | | |
| 80pg /ml | + + + + + + + + + + / + + + + + + + + + + / + + + + + + + + + + | | | | | | | + + + + + + + + + + / + + + + + + + + + + / + + + + + + + + + + | | | | | | |

| 160 pg/ml (Recombinant protein control product for target pathogens) | + | + | + | + | + | + | + | + | + | + | + | + | + | + | + | + | + | + | + |
| --- | --- | --- | --- | --- | --- | --- | --- | --- | --- | --- | --- | --- | --- | --- | --- | --- | --- | --- | --- |
| | + | + | + | + | + | + | + | + | + | + | + | + | + | + | + | + | + | + | + |
| | + | + | + | + | + | + | + | + | + | + | + | + | + | + | + | + | + | + | + |

Noted: "-" means no color development; "+" indicates color development intensities of different test lines, and the more the "+", the darker the color of the test line.

As could be seen from results of Table 3 that the flow of the solution was slow due to poor hydrophilicity of the sample pad. After the sample was added dropwise into the sample well of the test strip card for detection, the sample pad was located below the sample well, the lack of hydrophilicity of the sample pad led to the obstruction of the flow of the solution, and the solution accumulates on the sample pad and spread slowly, resulting in uneven sample flow, unilateral chromatography and slow chromatography. In addition, the sample pad treatment solution before optimization contained an excessive concentration of a blocking agent (bovine serum albumin), leading to poor sensitivity.

**Comparative example 2**

A test strip card for detection was prepared according to a method in Example 1, the only difference was that the absence of the support of the base card under the sample pad in the middle of the H-shaped test strip for detection. The test strip card for detection prepared above was used for testing according to the method in Example 2, with results shown in Table 4.

**Table 4 Detection results**

| Comparative item | With base plate | | | | | | | | | | Without base plate | | | | | | | | | |
|---|---|---|---|---|---|---|---|---|---|---|---|---|---|---|---|---|---|---|---|---|
| Project efficiency | Novel coronavirus | Influenza A virus | Influenza B virus | Syncytial virus | Adenovirus | Mycoplasma pneumoniae | Parainfluenzae virus | Group A streptococcus | Rhinovirus | Streptococcus pneumoniae | Novel coronavirus | Influenza A virus | Influenza B virus | Syncytial virus | Adenovirus | Mycoplasma pneumoniae | Parainfluenzae virus | Group A streptococcus | Rhinovirus | Streptococcus pneumoniae |
| Sensitivity | Good | | | | | | | | | | Bad | | | | | | | | | |
| Flow | Smooth | | | | | | | | | | Slow | | | | | | | | | |

EP 4 779 307 A1

| of solution | | | | | | | | | | | | | | | | | | | | |
|---|---|---|---|---|---|---|---|---|---|---|---|---|---|---|---|---|---|---|---|---|
| Negative matrix | - | - | - | - | - | - | - | - | - | - | - | - | - | - | - | - | - | - | - | - |
| 10pg/ml | + | + | + | + | + | + | + | + | + | + | + | - | - | + | - | - | - | - | - | - |
| 20 pg/ml | + + | + + | + + | + + | + + | + + | + + | + + | + + | + + | + | + | + | + | + | + | + | + | + | + |
| 40 pg/ml | + + | + + | + + | + + | + + | + + | + + | + + | + + | + + | + + | + + | + + | + + | + + | + + | + + | + + | + + | + + |
| 80pg/ml | + + + | + + + | + + + | + + + | + + + | + + + | + + + | + + + | + + + | + + + | + + + | + + + | + + + | + + + | + + + | + + + | + + + | + + + | + + + | + + + |

| 160 pg/ml (Recombinant protein control product for target pathogens) | | | | | | | | | | | | | | | | | | | |
|---|---|---|---|---|---|---|---|---|---|---|---|---|---|---|---|---|---|---|---|
| + | + | + | + | + | + | + | + | + | + | + | + | + | + | + | + | + | + | + | + |
| + | + | + | + | + | + | + | + | + | + | + | + | + | + | + | + | + | + | + | + |
| + | + | + | + | + | + | + | + | + | + | + | + | + | + | + | + | + | + | + | + |

Noted: "-" means no color; "+" indicates color development intensities of different detection lines, and the more the "+", the darker the color of the detection line.

As could be seen from above results that only a thin layer of sample pad placed in the middle of H-type test strip for detection could not ensure enough solution to flow left and right to the test strip. As the glass fiber member/polyester membrane was thin, after adding the sample dropwise, the solution remained at the bottom of the glass fiber member/-polyester membrane to cause the decrease of the sample size flowing to the test strip for chromatography, which led to the decrease of the sensitivity of the detection results and affects the accuracy and reliability of the detection results.

For this problem, a PVC base card in fit with the size of the sample pad is added at the bottom of the middle sample pad of the H-shaped test strip for detection. The size of the small PVC base card is 7 mm×4 mm, the size of the sample pad in the middle of the H shape is 18 mm × 4 mm, the small base card is stuck in the middle right below the sample pad, and the extra parts at the left and right ends of the sample pad are just overlapped on the test strips at the left and right ends. The adding of the small PVC base card at the bottom of the sample pad not only can effectively reduce the amount of solution by preventing excessive solution from gathering at the bottom of the sample pad, but also can make the overall structure of the test strip card for detection more stable. The small PVC base card provides a stable support platform for the sample pad in the middle of the H shape, such that the sample can flow to the left and right test strips more smoothly, and the flow of the solution can be effectively improved.

**Comparative example 3**

The test strip card for detection was prepared according to the method in Example 1, and the only difference was that the height of the boss in the cover plate of the housing was 2.2 mm, and the length from the boss close to the reaction zone to the nearest edge of the observation window was 1.3 mm. The test strip card for detection prepared above was used for detection according to the method of Example 2, with results shown in Table 5.

### Table 5 Detection results

| Comparative item | With boss and limiting adjustment | | | | | | | | | | Without boss and limiting adjustment | | | | | | | | | |
|---|---|---|---|---|---|---|---|---|---|---|---|---|---|---|---|---|---|---|---|---|
| Project efficiency | Novel coronavirus | Influenza A virus | Influenza B virus | Syncytial virus | Adenovirus | Mycoplasma pneumoniae | Parainfluenzae | Group A streptococcus pneumonia | Rhinovirus | Streptococcus | Novel coronavirus | Influenza A virus | Influenza B virus | Syncytial virus | Adenovirus | Mycoplasma pneumoniae | Parainfluenzae | Group A streptococcus pneumonia | Rhinovirus | Streptococcus |

| | virus | s | s | | | eumoniae | rus | coccus | neumoniae | irus | s | s | | | eumoniae | rus | coccus | neumoniae |
|---|---|---|---|---|---|---|---|---|---|---|---|---|---|---|---|---|---|---|
| Flow of solution | Smooth | | | | | | | | | Unilateral chromatography occured, and the test strips in the test zones of parainfluenza virus and rhinovirus were squeezed, and the flow of the solution was obstructed | | | | | | | |
| Negative matrix | - | - | - | - | - | - | - | - | - | - | - | - | - | - | - | / | - | / |
| 10pg /ml | + | + | + | + | + | + | + | + | + | + | + | + | + | + | + | / | + | / |
| 20pg /ml | ++ | ++ | ++ | ++ | ++ | ++ | ++ | ++ | ++ | ++ | + | + | + | + | + | / | + | / |
| 40 pg/ml | ++ | ++ | ++ | ++ | ++ | ++ | ++ | ++ | ++ | ++ | ++ | ++ | ++ | ++ | ++ | / | ++ | / |
| 80pg /ml | +++ | +++ | +++ | +++ | +++ | +++ | +++ | +++ | +++ | +++ | +++ | +++ | +++ | +++ | +++ | / | +++ | / |

| 160 pg/ml (Recombinant protein control product for target pathogens) | + | + | + | + | + | + | + | + | + | + | + | + | + | + | + | | + | | + |
|---|---|---|---|---|---|---|---|---|---|---|---|---|---|---|---|---|---|---|---|
| | + | + | + | + | + | + | + | + | + | + | + | + | + | + | + | / | + | / | + |
| | + | + | + | + | + | + | + | + | + | + | + | + | + | + | + | | + | | + |

Noted: "-" means no color development; "+" indicates color development intensities of different detection lines, the more the "+", the darker the color of the detection, and "/" means no testing.

As could be seen from results that if the boss was too high, the test strip would be severely squeezed, resulting in deformation of the flow channel and even the damage of the test strip. The height of the boss is 2.2 mm, and the height of the boss after adjusting is 1.85 mm. In Example 1, a position of the boss closest to the observation window was moved 0.5 mm away from the observation window, which was beneficial to reduce the local pressure of the boss on the nitrocellulose membrane, thus avoiding inaccurate detection results caused thereby. This indicated that the structural design of the housing of the test strip card for detection was unreasonable, the pressure distribution was uneven, the test strip was squeezed during the fixing process, the flow channel was deformed, and the solution did not flow smoothly, leading to poor repeatability of the detection results.

**Comparative example 4**

A test strip card for detection was prepared according to the method in Example 1, and testing was carried out according to the method of Example 2, and the only difference was that the sample drop volume for each test strip card for detection was 3 drops (about 80 μL). The results showed that the sample size of 3 drops was far insufficient. The solution failed to flow after sampling, resulting in a lack of chromatography, which prevented the test strip card for detection from achieving a satisfactory chromatographic result.

In the present disclosure, the sample drop volume for the test strip card for detection was 6 drops (about 160 μL) during testing. The results showed that the sample could move at a constant speed on the test strip card, the reaction zone of the test strip was wetted sufficiently while increasing the migration speed of the sample, which made the antigen-antibody complex react sufficiently to avoid problems of signal weakening and false negative caused by insufficient sample size.

The foregoing descriptions are merely preferred embodiments of the present disclosure. It should be noted that, for those of ordinary skill in the art, various modifications and embellishments can be made without departing from the principle of the present disclosure. Such modifications and embellishments shall be regarded as falling into the scope of protection of the present disclosure.

**Claims**

1. A combined immunochromatographic test strip for detection of multiple respiratory pathogenic microorganisms, comprising a test strip 1 and a test strip 2 which are both placed vertically, and a horizontally placed sampling strip;

    wherein a middle part of each of the test strip 1 and the test strip 2 is provided with a sampling zone, and with the sampling zone as the center, conjugate zones, reaction zones and absorption zones are sequentially and symmetrically overlapped above and below the sampling zone; one end of the sampling strip is overlapped with the sampling zone of the test strip 1, and the other end of the sampling strip is overlapped with the sampling zone of the test strip 2; and

    each conjugate zone is loaded with at least one primary antibody labeled with latex microspheres; each reaction zone is provided with at least one test line and a control line; the test line is coated with a secondary antibody; the primary antibody and the secondary antibody are able to specifically bind to the same respiratory pathogenic microorganism.

2. The combined immunochromatographic test strip for detection of multiple respiratory pathogenic microorganisms according to claim 1, wherein the test strip 1 is overlapped with a first absorbent zone, a first reaction zone, a first conjugate zone, a first sampling zone, a second conjugate zone, a second reaction zone, and a second absorbent zone in turn from top to bottom;

    wherein the test strip 2 is overlapped with a third absorbent zone, a third reaction zone, a third conjugate zone, a second sampling zone, a fourth conjugate zone, a fourth reaction zone and a fourth absorbent zone in turn from top to bottom;
    the first conjugate zone, the second conjugate zone, the third conjugate zone and the fourth conjugate zone are loaded with different types of primary antibodies against respiratory pathogenic microorganisms labeled with latex microspheres, respectively; and
    each of the first reaction zone, the second reaction zone, the third reaction zone and the fourth reaction zone is provided with a control line and test lines, wherein a number of the test lines is the same as that of primary antibodies loaded on the conjugate zone at the same end of the test strip; and each test line is coated with a secondary antibody against the respiratory pathogenic microorganism.

3. The combined immunochromatographic test strip for detection of multiple respiratory pathogenic microorganisms according to claim 2, wherein on each conjugate zone, a loading mass concentration of the primary antibody against the respiratory pathogenic microorganism loaded with the latex microsphere is independently 0.3%-0.4%.

4. The combined immunochromatographic test strip for detection of multiple respiratory pathogenic microorganisms according to claim 2, wherein a particle size of the latex microsphere ranges from 300 nm to 400 nm.

5. The combined immunochromatographic test strip detection of for multiple respiratory pathogenic microorganisms according to claim 2, wherein on each test line, a coating concentration of secondary antibody against the respiratory pathogenic microorganism is independently 0.5-2.0 mg/mL.

6. The combined immunochromatographic test strip for detection of multiple respiratory pathogenic microorganisms according to claim 2, wherein each control line is coated with goat anti-mouse IgG.

7. The combined immunochromatographic test strip for detection of multiple respiratory pathogenic microorganisms according to claim 6, wherein a coating concentration of the goat anti-mouse IgG ranges from 0.8 mg/mL to 1.5 mg/mL.

8. The combined immunochromatographic test strip for detection of multiple respiratory pathogenic microorganisms according to claim 2, wherein a sampling pad in the sampling zone on each of the test strip 1 and the test strip 2 and a sampling pad on the sampling strip are pre-treated with a first treatment solution;

wherein the first treatment solution is an aqueous solution containing 0.5% to 1% by mass of polyvinylpyrrolidone, 0.5% to 1% by mass of casein, 0.5% to 0.8% by volume of Tween-20, and 0.8% to 1.2% by mass of Tris.

9. The combined immunochromatographic test strip for detection of multiple respiratory pathogenic microorganisms according to any one of claims 2 to 8, wherein the first conjugate zone is coated with a primary antibody against influenza B virus labeled with blue latex microspheres, a primary antibody against influenza A virus labeled with blue latex microspheres, and a primary antibody against novel coronavirus labeled with red latex microspheres; the second conjugate zone is coated with a primary antibody against mycoplasma pneumoniae labeled with red latex microspheres, a primary antibody against respiratory adenovirus labeled with blue latex microspheres, and a primary antibody against respiratory syncytial virus labeled with blue latex microspheres; the third conjugate zone is coated with a primary antibody against rhinovirus labeled with blue latex microspheres, and a primary antibody against parainfluenza virus labeled with red latex microspheres; and the fourth conjugate zone is coated with a primary antibody against group A streptococcus labeled with red latex microspheres, and a primary antibody against streptococcus pneumoniae labeled with blue latex microspheres;

the first reaction zone is provided with three test lines and one first control line, the three test lines are coated with a secondary antibody against influenza B virus, a secondary antibody against influenza A virus, and a secondary antibody against novel coronavirus, respectively;

the second reaction zone is provided with three test lines and one second control line, the three test lines are coated with a secondary antibody against mycoplasma pneumoniae, a secondary antibody against respiratory adenovirus, and a secondary antibody against respiratory syncytial virus, respectively;

the third reaction zone is provided with two test lines and one third control line, and the two test lines are coated with a secondary antibody against rhinovirus, and a secondary antibody against parainfluenza virus, respectively; and

the fourth reaction zone is provided with two test lines and one fourth control line, and the two test lines are coated with a secondary antibody against group A streptococcus, and a secondary antibody against streptococcus pneumoniae, respectively.

10. A combined immunochromatographic test strip card for detection of multiple respiratory pathogenic microorganisms, comprising the combined immunochromatographic test strip for multiple respiratory pathogenic microorganisms according to any one of claims 1 to 9, a card slot base plate, and a cover plate capable of being snapped onto the card slot base plate;

a shape of a card slot on the card slot base plate is in fit with a structure of the test strip for detection to fix the test strip;

the cover plate is provided with a hollow sample well and a plurality of observation windows;

the sample well is located above the sampling strip; an observation window is arranged above each reaction zone in the test strip for detection for observing a test result.

11. The combined immunochromatographic test strip card for detection of multiple respiratory pathogenic microorganisms according to claim 10, wherein a plurality of linear bosses are also distributed on a back side of the cover plate; and the linear bosses are vertically distributed between the two observation windows visually.

12. The combined immunochromatographic test strip card for detection of multiple respiratory pathogenic microorganisms according to claim 11, wherein the number of the linear bosses is 2-4.

13. The combined immunochromatographic test strip card for detection of multiple respiratory pathogenic microorganisms according to claim 11, wherein a housing formed by snapping the cover plate onto the card slot base plate has an overall length of 118 mm, and a width of 35.7 mm.

14. The combined immunochromatographic test strip card for detection of multiple respiratory pathogenic microorganisms according to claim 13, wherein the cover plate has a height of 2.1 mm; the linear boss on the back side of the cover plate has a height of 1.85 mm, and a length from the linear boss close to the reaction zone to a nearest edge of the observation window is 1.8 mm.

15. A preparation method for the combined immunochromatographic test strip card for detection of multiple respiratory pathogenic microorganisms according to any one of claims 10 to 14, comprising the following steps:

pretreating a sampling pad in a sampling zone in each of a test strip 1 and a test strip 2 as well as a sampling pad on a sampling strip with a first treatment solution, and drying to obtain pre-treated sampling pads, wherein the first treatment solution is an aqueous solution containing 0.5% to 1% by mass of polyvinylpyrrolidone, 0.5% to 1% by mass of casein, 0.5% to 0.8% by volume of Tween-20, and 0.8% to 1.2% by mass of Tris;

spraying primary antibodies against different respiratory pathogenic microorganisms labeled with latex microspheres of different colors on a conjugate pad directly or in a hybrid manner, and drying to obtain a conjugate pad loaded with the primary antibodies labeled with the latex microspheres;

respectively spraying second antibodies against different types of respiratory pathogenic microorganisms on a nitrocellulose membrane to form test lines, and spraying mouse anti-IgG on the nitrocellulose membrane to form a control line, thus obtaining the nitrocellulose membrane coated with different second antibodies;

overlapping and assembling the sampling pad, the conjugate pad loaded with the primary antibodies labeled with the latex microspheres, the nitrocellulose membrane coated with different second antibodies and an absorbent pad to obtain a test board for detection, and cutting the test board for detection into strips to obtain test strips for detection; and

placing the test strips for detection on a card slot base plate, overlapping one end of a sampling strip on a sampling zone of the test strip 1, and the other end of the sampling strip on a sampling zone on the test strip 2, and snapping on a cover plate to obtain a test strip card for detection.

16. The preparation method according to claim 15, wherein the conjugate pad is pretreated with a second treatment solution,

wherein the second treatment solution is an aqueous solution containing the following components: 8%-12% by mass of 3-(N-morpholino)-2-hydroxypropanesulfonic acid, 4%-6% by mass of casein, 0.4%-0.6% by volume of Tween-20, and 0.9%-1.1% by volume of Proclin 300.

17. Use of the test strip for detection according to any one of claims 1 to 9, the combined immunochromatographic test strip card for detection of multiple respiratory pathogenic microorganisms according to claim 10, and a combined immunochromatographic test strip card for multiple respiratory pathogenic microorganisms prepared by the preparation method according to any one of claims 11 to 16 in detection of multiple respiratory pathogenic microorganisms or diagnosing diseases caused by the multiple respiratory pathogenic microorganisms.

18. The use according to claim 17, wherein the multiple respiratory pathogenic microorganisms comprise at least four of the following respiratory pathogenic microorganisms: influenza B virus, influenza A virus, novel coronavirus, mycoplasma pneumoniae, respiratory adenovirus, respiratory syncytial virus, rhinovirus, parainfluenza virus, streptococcus pneumoniae, and group A streptococcus.

**FIG. 1**

**FIG. 2**

FIG. 3

Applying sample here

Absorbent paper

NC membrane

(Conjugation pad)
polyester membrane

(Sample pad)
glass fiber

Sample pad with
base plate
attached

(Conjugation pad)
polyester membrane

NC membrane

Absorbent paper

**FIG. 4**

**TRANSLATION**

### INTERNATIONAL SEARCH REPORT

| International application No. |
|---|
| **PCT/CN2024/143209** |

| | |
|---|---|
| **A.** | **CLASSIFICATION OF SUBJECT MATTER** |
| | G01N 33/543(2006.01)i;  G01N 33/569(2006.01)i;  G01N 33/577(2006.01)i |

According to International Patent Classification (IPC) or to both national classification and IPC

| | |
|---|---|
| **B.** | **FIELDS SEARCHED** |

Minimum documentation searched (classification system followed by classification symbols)

IPC:G01N

Documentation searched other than minimum documentation to the extent that such documents are included in the fields searched

Electronic data base consulted during the international search (name of data base and, where practicable, search terms used)

CNTXT, ENTXTC, CNKI, web of science: 无锡白泰克, 李鹏程, 曾小琼, 周志图, 联合, 多联, 试纸, 免疫层析, 乳胶微球, 呼吸道, 多色, 颜色, 红色, 蓝色, 微生物, 腺病毒, 甲型流感, 甲流, 乙型流感, 乙流, 肺炎支原体, 合胞病毒, 质控线, 羊抗鼠, 聚乙烯吡咯烷酮, 酪蛋白, 吐温, 抗体, multi-test, multiplex, kit, immunochromatography, strip, respiratory, pathogens, Tween, Tris, ADV, RSV, HPIV, red, blue, fluorescent microspheres, antibody

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | CN 106053803 A (ANBIO (XIAMEN) BIOTECHNOLOGY CO., LTD.) 26 October 2016 (2016-10-26)<br>claims 1-10, description, paragraphs [0060]-[0106], and figures 1-3 | 1-5, 10-14, 17, 18 |
| Y | CN 106053803 A (ANBIO (XIAMEN) BIOTECHNOLOGY CO., LTD.) 26 October 2016 (2016-10-26)<br>claims 1-10, description, paragraphs [0060]-[0106], and figures 1-3 | 6-9, 15, 16 |
| Y | CN 112362869 A (SHANDONG KANGHUA BIOMEDICAL TECHNOLOGY CO., LTD.) 12 February 2021 (2021-02-12)<br>claims 1-10 | 6-8, 15, 16 |
| Y | CN 117434260 A (ASSURE TECH (HANGZHOU) CO., LTD.) 23 January 2024 (2024-01-23)<br>claims 1-10 | 9 |

| | | | |
|---|---|---|---|
| ✓ Further documents are listed in the continuation of Box C. | | ✓ See patent family annex. | |

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 July 2025** | **05 August 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/143209** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 116819081 A (SHANDONG KANGHUA BIOMEDICAL TECHNOLOGY CO., LTD. et al.) 29 September 2023 (2023-09-29)<br>entire document | 1-18 |
| A | CN 117434262 A (YOUKANG HOUDE BIOLOGICAL TECHNOLOGY (BEIJING) CO., LTD.) 23 January 2024 (2024-01-23)<br>entire document | 1-18 |
| A | CN 222014219 U (ASSURE TECH (HANGZHOU) CO., LTD.) 15 November 2024 (2024-11-15)<br>entire document | 1-18 |
| A | US 2024241117 A1 (WANG JINCHENG et al.) 18 July 2024 (2024-07-18)<br>entire document | 1-18 |
| A | CN 219758269 U (SHENZHEN ZHUORUN BIOTECHNOLOGY CO., LTD.) 26 September 2023 (2023-09-26)<br>entire document | 1-18 |

Form PCT/ISA/210 (second sheet) (July 2022)

**INTERNATIONAL SEARCH REPORT**
**Information on patent family members**

International application No.

**PCT/CN2024/143209**

| Patent document cited in search report | | | Publication date (day/month/year) | Patent family member(s) | | | Publication date (day/month/year) |
|---|---|---|---|---|---|---|---|
| CN | 106053803 | A | 26 October 2016 | None | | | |
| CN | 112362869 | A | 12 February 2021 | CN | 112362869 | B | 18 May 2021 |
| CN | 117434260 | A | 23 January 2024 | None | | | |
| CN | 116819081 | A | 29 September 2023 | None | | | |
| CN | 117434262 | A | 23 January 2024 | None | | | |
| CN | 222014219 | U | 15 November 2024 | None | | | |
| US | 2024241117 | A1 | 18 July 2024 | EP | 4403916 | A1 | 24 July 2024 |
| | | | | AU | 2023200795 | A1 | 01 August 2024 |
| CN | 219758269 | U | 26 September 2023 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202411737690